# EUROPÄISCHE PATENTSCHRIFT

(11) **EP 2 513 034 B1**
(45) Veröffentlichungstag und Bekanntmachung des Hinweises auf die Patenterteilung: **30.07.2014**
(21) Anmeldenummer: 10788085.8
(22) Anmeldetag: 13.12.2010
(51) Int. Cl.: C07C 201/08, C07C 205/06, C07C 205/12

(54) **SAURE SCHLAMMADSORPTION VON DNT-ABWÄSSERN**
ACID SLUDGE ADSORPTION OF DNT WASTE WATER
ADSORPTION DES BOUES ACIDES D'EAUX USÉES DINITROTOLUÈNE

(30) Priorität: 16.12.2009 EP 09179505
(43) Veröffentlichungstag der Anmeldung: 24.10.2012
(73) Patentinhaber: BASF SE, 67056 Ludwigshafen (DE)
(72) Erfinder: FRITZ, Rüdiger, 02994 Bernsdorf (DE); HEMPEL, Renate, 01945 Ruhland (DE); ZÖLLINGER, Michael, 73054 Eislingen (DE); MERTEN, Anne-Kathrin, 01979 Lauchhammer (DE); ZSCHIESCHANG, Doris, 01945 Guteborn (DE); HOMANN, Ina, 01968 Senftenberg (DE); GERSDORF, Annett, 01945 Hermsdorf (DE); ALLARDT, Holger, 01987 Schwarzheide (DE); REETZ, Reiner, 01987 Schwarzheide (DE); FLEGEL, Elvira, 02994 Bernsdorf (DE)
(86) Internationale Anmeldenummer: PCT/EP2010/069519
(87) Internationale Veröffentlichungsnummer: WO 2011/082974

(56) Entgegenhaltungen:
- EP-A1- 1 493 730
- EP-A1- 1 496 043
- DE-B- 1 221 994

## Beschreibung

Die vorliegende Erfindung betrifft ein Verfahren zur Aufarbeitung von alkalischem Prozessabwasser aus der Nitrierung von aromatischen Verbindungen, wobei das alkalische Prozessabwasser durch Zugabe von aufkonzentrierter Abfallschwefelsäure, die aus der Aufarbeitung der bei der Nitrierung anfallenden wässrigen, schwefelsäurehaltigen Phase stammt, angesäuert wird und das angesäuerte Prozessabwasser mit frischen Klärschlamm in Kontakt gebracht und der Klärschlamm anschließend abgetrennt wird.

Aromatische Nitroverbindungen wie Mono- und Dinitrotoluol werden üblicherweise durch Nitrierung der entsprechenden aromatischen Verbindungen mittels einer Mischung aus konzentrierter Salpetersäure und konzentrierter Schwefelsäure, die als Nitriersäure bezeichnet wird, hergestellt. Dabei entsteht eine organische Phase, die das Rohprodukt der Nitrierung enthält, und eine wässrige Phase, die im Wesentlichen Schwefelsäure, Reaktionswasser und durch die Nitriersäure eingetragenes Wasser enthält. Die Salpetersäure wird fast vollständig bei der Nitrierung verbraucht.

Nach Trennung der beiden Phasen wird die wässrige, schwefelsäurehaltige Phase je nach Technologie des Nitrierverfahrens direkt oder nach Aufkonzentrierung wieder mit frischer Salpetersäure gemischt und zur Nitrierung eingesetzt. Mindestens ein Teil der Schwefelsäure muss jedoch kontinuierlich oder diskontinuierlich aus dem Gesamtprozess ausgeschleust werden, um eine Aufkonzentration von Verunreinigungen, insbesondere metallischer Salze, zu vermeiden (siehe auch DE 10 143 800 C1). Bei den Verunreinigungen handelt es sich beispielsweise um ursprünglich in der Salpetersäure enthaltene Verunreinigungen und um Metallverbindungen, die unter den bei der Reaktion und der Aufarbeitung der wässrigen Phase herrschenden, stark korrosiven Bedingungen aus den Reaktor- und Rohrmaterialen herausgelöst werden.

Bei der Aufkonzentrierung der bei der Nitrierung anfallenden wässrigen schwefelsäurehaltigen Phase werden ein wässriges Destillat mit niedrigem Schwefelsäuregehalt, im Folgenden als wässriges Destillat der Schwefelsäureaufkonzentration bezeichnet, und eine Phase mit hohem Schwefelsäuregehalt, im Folgenden aufkonzentrierte Schwefelsäure genannt, erhalten. Der aus dem Nitrierverfahren ausgeschleuste Teil der aufkonzentrierten Schwefelsäure wird im Folgenden auch Abfallschwefelsäure genannt.

Das Rohprodukt der Nitrierung von aromatischen Verbindungen wie Benzol, Toluol, Xylol, Chlorbenzol etc. zu den entsprechenden Nitroaromaten enthält üblicherweise neben den gewünschten Nitroaromaten wie Nitrobenzol (NB) und Dinitrobenzol (DNB), Mono- und Dinitrotoluol (MNT und DNT), Nitrochlorbenzol (NCB) oder Nitroxylol auch geringe Mengen Mono-, Di- und Trinitrophenole (im Folgenden Nitrophenole genannt), Mono-, Di- und Trinitrokresole (im Folgenden Nitrokresole genannt) und Mono-, Di- und Trinitroxylenole (im Folgenden Nitroxylenole genannt) und andere Hydroxylgruppen und Nitrogruppen enthaltende Verbindungen sowie Mono- und Dinitrobenzoesäuren (im Folgenden Nitrobenzoesäuren genannt).

Aromatische Nitroverbindungen, die keine Hydroxylgruppe oder Carboxylgruppe im Molekül enthalten, werden im Rahmen der Erfindung auch als neutrale Nitroaromaten oder neutrale Nitrokörper bezeichnet. Nitrophenole, Nitrokresole, Nitroxylenole und Nitrobenzoesäuren werden im Folgenden auch als Hydroxynitroaromaten zusammengefasst.

Das Rohprodukt aus der Nitrierung muss vor Weiterverwendung von den unerwünschten Nebenprodukten befreit werden. Üblicherweise werden die Nebenprodukte nach Abtrennung der Nitriersäure durch mehrstufige Wäsche mit saurer, alkalischer und neutraler Waschflüssigkeit abgetrennt. Wobei in der Regel in der angegebenen Reihenfolge gewaschen wird. Die alkalische Wäsche wird üblicherweise mit wässriger Natronlauge, einer wässrigen Bicarbonatlösung oder einer wässrigen Ammoniaklösung durchgeführt. Das dabei entstehende alkalische Prozessabwasser enthält Nitrophenole, Nitrokresole, Nitroxylenole und Nitrobenzoesäuren in Form ihrer wasserlöslichen Salze der eingesetzten Base. Sie liegen üblicherweise in einer Konzentration von 0,2 bis 2,5 Gew.-%, bezogen auf das alkalische Prozessabwasser vor. Das alkalische Prozessabwasser enthält auch bei der Nitrierung gebildete neutrale Nitrokörper, insbesondere Reaktionsprodukte. Darüber hinaus enthält die alkalische Wäsche auch einen großen Anteil der entstandenen anorganischen Salze. Das alkalische Prozessabwasser enthält daher in der Regel 500 bis 5000 ppm Nitrate, 500 bis 5000 ppm Nitrit und einige Hundert ppm Sulfat. Neutrale Nitrokörper sind im alkalischen Prozessabwasser, üblicherweise in einer Menge von mehreren 1000 ppm enthalten. Aus den Inhaltsstoffen ergibt sich ein chemischer Sauerstoffbedarf von 1000 bis 20000 mg/L.

Die Nitrophenole, Nitrokresole, Nitroxylenole, Nitrobenzoesäuren und vor allem ihre Salze sind intensiv gefärbt und stark umwelttoxisch. Außerdem sind die Nitrophenole und speziell ihre Salze in höheren Konzentrationen oder in Substanz Sprengstoffe und müssen vor Abgabe des Abwassers aus diesem entfernt und derart entsorgt werden, dass von ihnen keine Gefahr für die Umwelt ausgeht. Da die aromatischen Nitroverbindungen insgesamt bakterizide Eigenschaften aufweisen und somit eine biologische Reinigung des Abwassers unmöglich machen, ist eine Reinigung oder Aufarbeitung der aromatische Nitroverbindungen enthaltenden Abwässer notwendig.

Zur Entfernung der Nitrophenole, Nitrokresole, Nitroxylenole, Nitrobenzoesäuren und der neutralen Nitroaromaten aus den Prozessabwässern sind zahlreiche Verfahren in der Literatur beschrieben, beispielsweise Extraktion, Adsorption, Oxydation oder Thermolyse.

In der Encyclopedia of Chemical Technology, Kirk-Othmer, Fourth Edition 1996, Vol. 17, S. 138 wird ein Extraktionsverfahren zur Abtrennung von Nitrobenzol beschrieben, bei dem das im Abwasser bei der entsprechenden Temperatur gelöste Nitrobenzol durch Extraktion mit Benzol entfernt wird. Benzol, das sich im Wasser gelöst hat, wird vor der Endbehandlung des Abwassers durch Strippen entfernt.

Gemäß US-A 6,506,948 werden die bei der Nitrierung von Benzol anfallenden wässrigen Waschphasen direkt mit Toluol extrahiert, wobei jeder der entstehenden Abwasserströme separat extrahiert wird. Der Toluolstrom wird anschließend in den Nitrierprozess geführt und umgesetzt. Dabei bleiben Nitrokresole und Nitrobenzoesäuren im alkalischen Abwasserstrom gelöst und müssen anschließend separat entfrachtet werden.

Die gelösten Nitroaromaten und Hydroxynitroaromaten können weiterhin im sauren Milieu durch Extraktion mit einem organischen Lösungsmittel entfernt werden (Ullmanns Enzyklopädie der technischen Chemie, 4. Auflage, Band 17, Seite 386).

Die in dem alkalischen Prozessabwasser enthaltenen Hydroxynitroaromaten können auch durch Ansäuern in eine sich abscheidende organische Phase überführt werden, die anschließend abgetrennt wird. Ein derartiges Verfahren ist in EP 1 493 730 A1 beschrieben. Gemäß diesem Verfahren werden die Abwasserströme der neutralen und alkalischen DNT-Wäschen und der Schwefelsäureaufkonzentrierung vermischt, so dass sich ein pH-Wert unterhalb von 5 einstellt. Bei dem Abwasser der Schwefelsäureaufkonzentrierung handelt sich um das Destillat der Schwefelsäureaufkonzentrierung mit einer Schwefelsäurekonzentration von 0,2 bis 1 Gew.- %. Beim Ansäuern scheidet sich eine organische Phase ab, die abgetrennt wird. Die wässrige Phase wird separat einer weiteren Abwasseraufbereitung zugeführt. Um das Auskristallisieren der Hydroxynitroaromaten zu verhindern, muss die für das Abscheiden und Abtrennen verwendete Apparatur beheizt werden.

In EP 0 005 203 wird ein thermisches Verfahren zur Aufarbeitung von Hydroxynitroaromaten enthaltenden Abwässern beschrieben. Hierbei werden die Abwässer, die die Hydroxynitroaromaten in Form ihrer wasserlöslichen Salze enthalten, unter Luft- und Sauerstoffausschluss unter Druck auf Temperaturen im Bereich 150 bis 500°C erhitzt.

Aus EP 0 953 546 ist ein thermisches Verfahren zur Aufarbeitung von Abwasserströmen aus Nitrieranlagen, bei dem gleichzeitig Hydroxynitroaromaten und neutrale Nitroaromaten abgebaut werden können, bekannt.

Gemäß WO 2009/027416 A1 werden vor der thermolytischen Behandlung der alkalischen Abwässer aus der Nitrierung die darin gelösten, aromatischen Nitroverbindungen, die keine Hydroxylgruppen enthalten, durch Extraktion entfernt.

EP 1 496 043 A1 beschreibt ein Verfahren zur Aufarbeitung von wässrigen Abwässern, die aus der Nitrierung von Toluol zu Dinitrotoluol mit Nitriersäure anfallen. Die wässrigen Abwässer enthalten dabei saures Waschwasser, alkalisches Waschwasser, jeweils aus der Wäsche des Dinitrotoluols, sowie Destillat aus der Schwefelsäureaufkonzentrierung. Die Abwässer werden so vereinigt, dass sich ein pH-Wert von unter 5 einstellt, die entstehende wässrige und organische Phase werden durch Phasentrennung getrennt und die dabei erhaltene wässrige Phase wird mit Toluol extrahiert und dieses anschließend der Nitrierung zugeführt.

EP 1 493 730 A1 offenbart ein Verfahren zur Aufarbeitung von organischen Nebenkomponenten, die bei der Nitrierung von Toluol zu Dinitrotoluol entstehen. Dabei wird wie vorstehend für EP 1 496 043 A1 beschrieben vorgegangen, mit dem Unterschied, dass die bei der Phasentrennung der wässrigen und organischen Phase erhaltene organische Phase direkt in den Nitrierprozess zurückgeführt wird.

DE 12 21 994 B beschreibt ein Verfahren zur Reinigung von nitrokresolhaltigen Abwässern, die bei der alkalischen und sauren Wäsche des Rohprodukts der Nitrierung von Toluol stammen. Dazu werden die Abwässer nach Einstellung des pH-Werts auf 7 bis 9 mithilfe eines basischen Anionenaustauschers gereinigt, der anschließend mit einer Mineralsäure und einem organischen Lösungsmittel regeneriert wird.

Nachteilig an den vorstehend beschriebenen Verfahren ist dabei der oft relativ hohe energetische Aufwand einer Thermolyse oder einer Wasserdampfstrippung. Wird das alkalische Prozessabwasser auf einen sauren pH-Wert eingestellt und die sich dabei abscheidende organische Phase, die Hydroxynitroaromaten und Nitrobenzoesäuren enthält, lediglich mittels Phasentrennung abgetrennt, tritt das Problem des so genannten "Foulings" auf. Dies bedeutet, dass die zur Abtrennung der sich abscheidenden organischen Phase verwendeten Pumpen und Rohrsysteme sehr schnell durch ausfallende und auskristallisierende Verunreinigungen verstopfen und daher ein hoher Reinigungsbedarf besteht.

Weiterhin ist bekannt, bei der Dinitrotoluol-Herstellung das dinitrotoluolhaltige, alkalische Abwasser durch eine Absorption am Klärschlamm vom größten Teil des bei 70°C gelösten 2,4- und 2,6-Dinitrotoluol zu befreien, bevor die Hydroxynitroaromaten mittels Ozonisierung aufgeschlossen werden. Hierbei stellt sich das Problem, dass die Nitrokresole durch die Ozonierung nicht umgewandelt werden können. Zudem enthält das alkalische Abwasser noch Nitrit, das erst durch Ozon zu Nitrat oxidiert wird und so zu einem erhöhten Ozonbedarf führt. So werden bei einer Konzentration von 3000 mg/L Nitrit im alkalischen Prozessabwasser etwa 25 bis 40% des Ozons für die Nitrit-Oxidation verbraucht.

Daher besteht die Aufgabe der vorliegenden Erfindung darin, ein Verfahren bereitzustellen, das aus den alkalischen Prozessabwassern der Nitrierung von aromatischen Verbindung auch die Abtrennung der Nitrokresole ermöglicht und den Energie- und Kostenaufwand bei der weiteren Behandlung des Abwassers mit gebräuchlichen Aufbereitungsverfahren verringert.

Diese Aufgabe wird gelöst durch das folgende Verfahren zur Aufarbeitung von alkalischem Prozessabwasser aus der Nitrierung von aromatischen Verbindungen zu Mono-, Di- und Trinitroaromaten, das einen pH-Wert von 7,5 bis 13 aufweist, umfassend die Schritte
a) Ansäuern des alkalischen Prozessabwassers durch Zugabe von aufkonzentrierter Schwefelsäure, die aus der Aufarbeitung der bei der Nitrierung anfallenden wässrigen, schwefelsäurehaltigen Phase stammt, auf einen pH-Wert unterhalb von 5, wobei eine Mischung A aus sich abscheidender organischer Phase und saurer wässriger Phase entsteht,
b) In Kontaktbringen der Mischung A mit frischem Klärschlamm, und
c) Abtrennen des Klärschlamms.

Das mit dem erfindungsgemäßen Verfahren behandelte alkalische Prozessabwasser ist an schwerabbaubaren neutralen Nitrokörpern, Nitrokresolen und Nitrobenzoesäuren sowie an Nitrit stark verarmt. Typischerweise beträgt die prozentuale Erniedrigung des Gehalts dieser Verbindungen in einem erfindungsgemäß behandelten alkalischen Prozessabwasser aus einer Anlage zur Herstellung von Dinitrotoluol bei Einbeziehung der Verdünnung durch den Klärschlamm:

| | |
|---|---|
| 2,4- DNT: | 50 - 57%, |
| 2,6- DNT: | 50 - 80%, |
| Summe (Nitrokresole und Nitrobenzoesäure): | 70 - 90%, |
| Summe (Nitrit): | 85 - 95%. |

Ein weiterer Vorteil des erfindungsgemäßen Verfahrens besteht darin, dass für das Ansäuern des alkalischen Prozessabwassers die gesamte Menge der beim Säureaufkonzentrieren anfallenden Abfallschwefelsäure aus dem Herstellungsprozess der Nitroaromaten verwendet werden kann. Die aufkonzentrierte Schwefelsäure enthält die durch Korrosion (Rohrleitungen) bei der Nitrierung anfallenden Salze umfassend Fe, Cr, Ni, Ta, und Spuren weiterer Schwermetalle in Form ihrer Sulfate. Üblicherweise wird bei einem Anstieg der Salzkonzentration über 300 ppm ein Teil der Säure als so genannte Abfallschwefelsäure aus dem Prozess geschleust und muss entsorgt bzw. mit anderen Verfahren aufgereinigt werden. Somit ist die Verwendung dieser Abfallschwefelsäure besonders vorteilhaft, da man sich die Entsorgungs- bzw. Aufarbeitungskosten einsparen kann. Die Verwendung der aufkonzentrierten Abfallschwefelsäure führt weiterhin dazu, dass beim Ansäuern des alkalischen Prozessabwassers ein großer Teil des im alkalischen Prozessabwassers gelösten Nitrits zu salpetriger Säure protoniert wird, die dann in Salpetersäure und Stickoxide disproportioniert. Die Stickoxide können abgetrennt und beispielsweise zur Verwertung in der Salpetersäureherstellung eingesetzt werden. Gemäß einer bevorzugten Ausführungsform der Erfindung werden die sich beim Ansäuern abscheidenden Stickstoffoxide in die Salpetersäurerückgewinnung der Nitrieranlage eingespeist und gehen dem Prozess somit nicht verloren. Der chemische Sauerstoffbedarf des mit dem erfindungsgemäßen Verfahren behandelten Abwasserstroms wird deutlich verringert. Durch die Verwendung der aufkonzentrierten Abfallschwefelsäure wird die Menge an zu reinigendem Prozessabwasser nicht unnötig vergrößert, wie dies bei Verwendung von verdünnter Säure der Fall ist.

Das erfindungsgemäße Verfahren wird zur Aufarbeitung von alkalischem Prozessabwasser aus der Nitrierung von aromatischen Verbindungen eingesetzt. Vorzugsweise handelt es sich bei den aromatischen Verbindungen um Benzol, Toluol, Xylol, Chlorbenzol und/oder Dichlorbenzol.

Das aus der ein- bzw. mehrstufige Wäsche des Rohprodukts der Nitrierung mit wässriger Natronlauge, wässriger Carbonat- oder Hydrogencarbonatlösung oder wässriger Ammoniaklösung erhaltene alkalische Prozessabwasser weist in Abhängigkeit von der eingesetzten Base einen pH-Wert von 7,5 bis 13, bevorzugt 8 bis 10 (gemessen bei 60°C) auf.

Erfindungsgemäß wird das alkalische Prozessabwasser durch Zugabe von aufkonzentrierter Schwefelsäure, die aus der Aufarbeitung der bei der Nitrierung anfallenden, wässrigen schwefelsäurehaltigen Phase stammt, auf einem pH-Wert unterhalb von 5, bevorzugt von 0,5 bis 3 eingestellt. Die pH-Wert-Angaben beziehen sich jeweils auf die Messung bei 60°C. Beim Ansäuern des alkalischen Prozessabwassers scheidet sich eine organische Phase ab, die Hydroxynitroaromaten, Nitrobenzoesäuren und neutrale Nitrokörper enthält. Das angesäuerte, ursprünglich alkalische Prozessabwasser mit der sich abscheidenden organischen Phase wird im Rahmen der Erfindung als Mischung A bezeichnet.

Die zur Ansäuerung eingesetzte Abfallschwefelsäure weist eine Konzentration von 85 bis 95 Gew.-%, bevorzugt von 90 bis 93 Gew.-% auf. Gemäß einer bevorzugten Ausführungsform wird nur bei der Nitrierung anfallende Abfallschwefelsäure zum Ansäuern in Schritt a) zugegeben, besonders bevorzugt wird die gesamte bei der Nitrierung anfallende Abfallschwefelsäure in Schritt a) zugegeben. Die Steuerung der Zugabe der aufkonzentrierten Schwefelsäure erfolgt in vorteilhafter Weise über eine Online-pH-Messung.

Das alkalische Prozessabwasser fällt üblicherweise bei einer Temperatur von 55 bis 80°C an. Erfindungsgemäß wird das alkalische Prozessabwasser auch bei dieser Temperatur durch Zugabe der aufkonzentrierten Schwefelsäure angesäuert. Die Vermischung der aufkonzentrierten Schwefelsäure mit dem alkalischen Prozessabwasser führt zu einer starken Gasentwicklung. Das sich abscheidende Gasgemisch enthält NOₓ, insbesondere Stickstoffmonoxid, Stickstoffdioxid und Distickstoffmonoxid. Wurde als alkalisches Waschwasser Carbonat- und/oder Hydrogencarbonatlösung eingesetzt, enthält das sich abscheidende Gas üblicherweise 70 bis 98,9 Vol.-% Kohlendioxid und 1,1 bis 30 Vol.-% nitrose Gase (NOx). Bevorzugt enthält das sich abscheidende Gasgemisch 80 bis 98 Vol.-% Kohlendioxid und 2 bis 20 Vol.-% nitrose Gase. Enthält das Prozessabwasser anstatt Alkalicarbonat- und/oder -hydrogencarbonat eine andere Base, die nach dem Ansäuern keine gasförmige Komponente bildet, besteht das Abgas ausschließlich aus NOₓ, üblicherweise 47 bis 98% Stickstoffmonoxid, 1 bis 47% Stickstoffdioxid und 1 bis 6 % Distickstoffmonoxid. Bevorzugt werden die sich beim Ansäuern abscheidenden NOₓ-haltigen Abgase abgetrennt und zur Verwertung in der Salpetersäureherstellung eingesetzt. Besonders bevorzugt werden die abgetrennten Stickoxide in die Salpetersäurerückgewinnung der Nitrieranlage zurückgeführt. Dabei wird das Gasgemisch üblicherweise in die Absorptionskolonnen der Stickstoffsmonoxid- und Stickstoffdioxid- Absorption der Salpetersäurerückgewinnung der Nitrieranlagen eingespeist. Besonders vorteilhaft ist, wenn das gesamte Gasgemisch direkt und ohne vorherige Abtrennung von CO₂ und Reinigung rückgeführt wird.

Als Absorptionsmittel wird in Schritt b) des erfindungsgemäßen Verfahrens frischer Klärschlamm eingesetzt. "Frisch" bedeutet in diesem Zusammenhang, dass der Klärschlamm aus einer Kläranlage stammt und noch nicht zur Absorption im erfindungsgemäßen Verfahren oder einem ähnlichen Verfahren eingesetzt wurde. Erfindungsgemäß bevorzugt wird biologisch aktiver Klärschlamm verwendet, der noch solange aktiv ist, dass der restliche Anteil des im Wasser gelösten Nitrits zu Nitrat oxidiert werden kann. Auf dieser Weise kann ein zusätzlicher positiver Effekt erzielt werden, da das Nitrit bei einer anschließenden Weiterbehandlung stören kann, wenn zum Beispiel bei der Ozonbehandlung zunächst Nitrit zu Nitrat oxidiert werden muss und so unnötig Ozon verbraucht wird.

Üblicherweise weist der erfindungsgemäß als Absorptionsmittel eingesetzte Klärschlamm einen Feststoffanteil von 10 - 25 g/l auf.

Erfindungsgemäß werden der Klärschlamm und die Mischung A für eine Minute bis zu zwei Tage miteinander in Kontakt gebracht, bevorzugt für 2 Minuten bis 1,5 Tage.

Erfindungsgemäß bevorzugt werden der Klärschlamm und die Mischung A dadurch miteinander in Kontakt gebracht, dass sie in einem gemeinsamen Behälter geführt werden. Weiter bevorzugt werden die Mischung A und der Klärschlamm in Schritt b) mit Hilfe von aktiven und/oder passiven Mischelementen in Kontakt gebracht. Geeignete aktive und passive Mischelemente sind dem Fachmann bekannt. Unter aktiven Mischelementen sind beispielsweise Rührer, Ultraschall, Schüttler aufzuzählen. Als statische Mischelemente können beispielsweise Kanäle und Einbauten verwendet werden.

Das alkalische Prozessabwasser fällt üblicherweise bei einer Temperatur von 50 bis 80°C an. Erfindungsgemäß wird das alkalische Prozessabwasser auch bei dieser Temperatur durch Zugabe der aufkonzentrierten Abfallschwefelsäure angesäuert (Schritt a)). Die Mischung A weist zu Beginn bei in Kontaktbringen mit dem Klärschlamm (Schritt b)) eine Temperatur von 50 bis 80°C auf. Erfindungsgemäß bevorzugt liegt die Temperatur des alkalischen Prozessabwassers in Schritt a) und der Mischung A zu Beginn des Schritts b) bei 60 bis 75°C.

Der Klärschlamm weist zu Beginn von Schritt b) bei in Kontaktbringen mit der Mischung A eine Temperatur von 5 bis 25°C, bevorzugt von 8 bis 15°C auf. Dies hat den Vorteil, dass durch den Abkühlungseffekt und den Feststoffanteil am Klärschlamm, die bei Ansäuerung des alkalischen Prozessabwassers sich abscheidende organische Phase ausfällt und an dem Klärschlamm absorbiert. In einer bevorzugten Ausführungsform der Erfindung wird das Volumenverhältnis von Klärschlamm zur Mischung A so gewählt, dass sich eine Temperatur von 30°C oder weniger einstellt. Durch die starke Abkühlung fallen die organischen Verbindungen, die einen Schmelzpunkt oberhalb von 30°C aufweisen, aus und scheiden sich auf den Feststoffbestandteilen des Klärschlamms ab.

Üblicherweise liegt das Volumenverhältnis von Klärschlamm zur Mischung A bei 5:1 bis 1:2 bevorzugt bei 3:1 bis 1:1.

Nach in Kontaktbringen des Klärschlamms mit der Mischung A in Schritt b) wird in Schritt c) der Klärschlamm abgetrennt. Dies wird mit Hilfe üblicher, dem Fachmann bekannten Vorrichtungen, beispielsweise mit Hilfe von Dekantern oder Zentrifugen durchgeführt, bevorzugt wird der Klärschlamm mit Hilfe von statischen Abscheidern abgetrennt.

Das erfindungsgemäße Verfahren kann batchweise oder kontinuierlich durchgeführt werden. Erfindungsgemäß bevorzugt wird das Verfahren kontinuierlich durchgeführt.

Im Folgenden wird das erfindungsgemäße Verfahren anhand von Beispielen näher erläutert:

### Beispiel 1

In einem 1000 mL Rührbehälter mit Gasabführung und Innenthermometer werden 400 mL eines alkalischen Prozessabwassers aus der Produktion von Dinitrotoluol bei 60°C vorgelegt. Die Konzentrationen an DNT, Nitrokresolen und Nitrobenzoesäuren sind in Tabelle 1 aufgeführt. Anschließend wird das Prozessabwasser mit 93%iger aufkonzentreirter Abfallschwefelsäure versetzt, bis ein pH-Wert von 2,5 erreicht wird. Das entstehende Gasgemisch wird über die Gasabführung in eine benachbarte Absorberkaskade geleitet, die mit 25%iger KOH gefüllt ist. Nachdem die Gasbildung beendet ist, werden 400 mL frischen Klärschlamms mit 15 °C aus der Biologie I der Kläranlage der BASF Schwarzheide GmbH zugegeben. Der Klärschlamm besitzt eine Trockensubstanz von 20 g/L. Dabei stellt sich eine Mischungstemperatur von 37°C ein. Anschließend wird die Mischung für 30 Minuten gerührt und in ein Vorlagegefäß umgefüllt. Nach Zentrifugieren wird der Überstand analysiert. Die Ergebnisse sind ebenfalls in Tabelle 1 dargestellt.

**Tabelle 1:**

| Komponente | Konzentration vor Behandeln [mg/L] | Konzentration nach Behandeln (erfindungsgemäß) [mg/L] | Mengenreduktion [%] |
|---|---|---|---|
| 2,4-DNT | 556 | 214 | 62 |
| 2,6-DNT | 132 | 42,0 | 68 |
| Nitrokresole, Nitrobenzoesäure | 541 | 77,6 | 86 |

### Beispiel 2

In einem 500 mL Rührbehälter (B1) mit Gasabführung zu einem Absorber (A1) und Innenthermometer werden kontinuierlich 1000 mL/h eines alkalischen Prozessabwassers aus der Produktion von Dinitrotoluol mit 60°C und kontinuierlich 93%ige aufkonzentrierte Abfallschwefelsäure aus der Säureaufkonzentrierung, gesteuert über eine Inline-pH-Wert-Messung, zugeführt. Der pH-Wert wird auf einen Wert von 0,5 eingestellt. Das sich bildende Gasgemisch wird über eine Gasabführung auf eine Absorptionskolonne (A1) geführt und so dem Prozess entzogen. Aus dem Rührbehälter (B1) werden kontinuierlich 525 mL des angesäuerten Abwassers von 65°C in den Behälter (B2) gefördert. In den Rührbehälter B2 werden kontinuierlich 525 mL/h des Klärschlamms der Biologie I der Kläranlage der BASF Schwarzheide GmbH mit 10°C eingebracht. Der Klärschlamm besitzt eine Trockensubstanz von 20 g/L. Aus dem 5000 mL Rührbehälter B2, der zu 70% gefüllt ist, wird kontinuierlich ein Volumenstrom von 1025 mL/h mit 25°C in einen Dekanter (D) gefördert, der nach Absetzen des Klärschlamms und der adsorbierten organischen Komponenten einen vorbehandelten Abwasserstrom mit 21°C liefert. Der Behälter B2 ist über eine Abgasleitung mit der Absorptionskolonne (A1) verbunden. Der Abwasserstrom aus dem Dekanter (D) wird analysiert. Die Konzentrationen an DNT, Nitrokresolen und Nitrobenzoesäuren in den behandelten und unbehandelten Abwässern sind in Tabelle 2 gegenübergestellt.

**Tabelle 2:**

| Komponente | Konzentration vor Behandeln [mg/L] | Konzentration nach Behandeln (erfindungsgemäß) [mg/L] | Mengenreduktion [%] |
|---|---|---|---|
| 2,4-DNT | 556 | 186 | 67 |
| 2,6-DNT | 132 | 38,6 | 71 |
| Nitrokresole, Nitrobenzoesäure | 541 | 50,8 | 91 |

### Beispiel 3

Beispiel 3 wird ausgeführt wie Beispiel 2 mit dem Unterschied, dass der pH-Wert, der durch die Dosierung von Abfallschwefelsäure aus der Säurenaufkonzentrierung über eine inline-Messung gesteuert wird, auf 2,5 eingestellt wird. Die Konzentration von DNT, Nitrokresolen und Nitrobenzoesäuren vor und nach dem erfindungsgemäßen Behandeln sind in Tabelle 3 gegenübergestellt.

**Tabelle 3:**

| Komponente | Konzentration vor Behandeln [mg/L] | Konzentration nach Behandeln (erfindungsgemäß) [mg/L] | Mengenreduktion [%] |
|---|---|---|---|
| 2,4-DNT | 556 | 264 | 53 |
| 2,6-DNT | 132 | 59,4 | 55 |
| Nitrokresole, Nitrobenzoesäure | 541 | 155 | 71 |

## Patentansprüche

1. Verfahren zur Aufarbeitung von alkalischem Prozessabwasser aus der Nitrierung von aromatischen Verbindungen zu Mono-, Di- und Trinitroaromaten, das einen pH-Wert von 7,5 bis 13 aufweist, umfassend die Schritte
a) Ansäuern des alkalischen Prozessabwassers durch Zugabe von aufkonzentrierter Schwefelsäure, die aus der Aufarbeitung der bei der Nitrierung anfallenden wässrigen, schwefelsäurehaltigen Phase stammt, auf einen pH-Wert unterhalb von 5, wobei eine Mischung A aus sich abscheidender organischer Phase und saurer wässriger Phase entsteht,
b) In Kontaktbringen der Mischung A mit frischem Klärschlamm, und
c) Abtrennen des Klärschlamms.

2. Verfahren nach Anspruch 1, **dadurch gekennzeichnet, dass** die zur Ansäuerung verwendete aufkonzentrierte Schwefelsäure eine Konzentration von 85 bis 95 Gew.-%, bevorzugt von 90 bis 93 Gew.-% aufweist.

3. Verfahren nach Anspruch 1 oder Anspruch 2, **dadurch gekennzeichnet, dass** die gesamte bei der Nitrierung anfallende Abfallschwefelsäure in Schritt a) eingesetzt wird.

4. Verfahren nach einem der Ansprüche 1 bis 3, **dadurch gekennzeichnet, dass** in Schritt a) der pH-Wert auf 0,5 bis 3 eingestellt wird.

5. Verfahren nach einem der Ansprüche 1 bis 4, **dadurch gekennzeichnet, dass** die Mischung A und der Klärschlamm für 1 Minute bis 2 Tage miteinander in Kontakt gebracht werden.

6. Verfahren nach einem der Ansprüche 1 bis 5, **dadurch gekennzeichnet, dass** es sich bei den aromatischen Verbindungen um Benzol, Toluol, Xylenol, Chlorbenzol und/oder Dichlorbenzol handelt.

7. Verfahren nach einem der Ansprüche 1 bis 6, **dadurch gekennzeichnet, dass** das alkalische Prozessabwasser in Schritt a) und die Mischung A zu Beginn von Schritt b) eine Temperatur von 50 bis 80 °C, bevorzugt von 60 bis 75 °C aufweist.

8. Verfahren nach einem der Ansprüche 1 bis 7, **dadurch gekennzeichnet, dass** der Klärschlamm zu Beginn von Schritt b) eine Temperatur von 5 bis 25 °C, bevorzugt von 8 bis 15 °C aufweist.

9. Verfahren nach einem der Ansprüche 1 bis 8, **dadurch gekennzeichnet, dass** in Schritt b) das Volumenverhältnis von Klärschlamm zu Mischung A so gewählt wird, dass sich eine Temperatur von 30 °C oder weniger einstellt.

10. Verfahren nach einem der Ansprüche 1 bis 9, **dadurch gekennzeichnet, dass** in Schritt b) das Volumenverhältnis von Klärschlamm zu Mischung A bei 5:1 bis 1:2, bevorzugt 3:1 bis 1:1 beträgt.

11. Verfahren nach einem der Ansprüche 1 bis 10, **dadurch gekennzeichnet, dass** die Mischung A und der Klärschlamm in Schritt b) mit Hilfe von aktiven und/oder passiven Mischelementen in Kontakt gebracht werden.

12. Verfahren nach einem der Ansprüche 1 bis 11, **dadurch gekennzeichnet, dass** die sich beim Ansäuern in Schritt a) abscheidenden Stickstoffoxide abgetrennt und zur Verwertung in die Salpetersäureherstellung eingesetzt werden.

13. Verfahren nach einem der Ansprüche 1 bis 12, **dadurch gekennzeichnet, dass** die sich beim Ansäuern in Schritt a) abscheidenden Stickstoffoxide abgetrennt und in die Salpetersäurerückgewinnung der Nitrieranlage rückgeführt werden.

14. Verfahren nach einem der Ansprüche 1 bis 13, **dadurch gekennzeichnet, dass** der Klärschlamm in Schritt c) in einem statischen Abscheider abgetrennt wird.

15. Verfahren nach einem der Ansprüche 1 bis 14, **dadurch gekennzeichnet, dass** das Verfahren in kontinuierlicher Fahrweise durchgeführt wird,

## Claims

1. A process for working up alkaline process wastewater from the nitration of aromatic compounds to mono-, di- and trinitroaromatics, said alkaline process wastewater having a pH of 7.5 to 13, comprising the steps of
a) acidifying the alkaline process wastewater by adding concentrated sulfuric acid which originates from the workup of the aqueous, sulfuric acid-containing phase obtained in the nitration to a pH below 5, which forms a mixture A consisting of organic phase which separates out and acidic aqueous phase,
b) contacting the mixture A with fresh sewage sludge, and
c) removing the sewage sludge.

2. The process according to claim 1, wherein the concentrated sulfuric acid used for acidification has a concentration of 85 to 95% by weight, preferably of 90 to 93% by weight.

3. The process according to claim 1 or claim 2, wherein all of the waste sulfuric acid obtained in the nitration is used in step a).

4. The process according to any of claims 1 to 3, wherein the pH is adjusted to 0.5 to 3 in step a).

5. The process according to any of claims 1 to 4, wherein the mixture A and the sewage sludge are contacted with one another for 1 minute to 2 days.

6. The process according to any of claims 1 to 5, wherein the aromatic compounds are benzene, toluene, xylenol, chlorobenzene and/or dichlorobenzene.

7. The process according to any of claims 1 to 6, wherein the alkaline process wastewater in step a) and the mixture A at the start of step b) has a temperature of 50 to 80°C, preferably of 60 to 75°C.

8. The process according to any of claims 1 to 7, wherein the sewage sludge at the start of step b) has a temperature of 5 to 25°C, preferably of 8 to 15°C.

9. The process according to any of claims 1 to 8, wherein the volume ratio of sewage sludge to mixture A in step b) is selected such that a temperature of 30°C or less is established.

10. The process according to any of claims 1 to 9, wherein the volume ratio of sewage sludge to mixture A in step b) is 5:1 to 1:2, preferably 3:1 to 1:1.

11. The process according to any of claims 1 to 10, wherein the mixture A and the sewage sludge in step b) are contacted with the aid of active and/or passive mixing elements.

12. The process according to any of claims 1 to 11, wherein the nitrogen oxides which separate out in the course of acidification in step a) are removed and utilized in nitric acid preparation.

13. The process according to any of claims 1 to 12, wherein the nitrogen oxides which separate out in the course of acidification in step a) are removed and recycled into the nitric acid recovery in the nitrating plant.

14. The process according to any of claims 1 to 13, wherein the sewage sludge is removed in step c) in a static separator.

15. The process according to any of claims 1 to 14, which is performed in continuous mode.

## Revendications

1. Procédé pour le traitement d'eau résiduaire alcaline de processus, provenant de la nitration de composés aromatiques conduisant à des composés mono-, di- et trinitroaromatiques, qui présente un pH de 7,5 à 13, comprenant les étapes
a) acidification de l'eau résiduaire alcaline de processus par addition d'acide sulfurique concentré, qui provient du traitement de la phase aqueuse, contenant de l'acide sulfurique, produite lors de la nitration, jusqu'à un pH inférieur à 5, de sorte qu'il en résulte un mélange A de phase organique et phase aqueuse acide qui se séparent,
b) mise en contact du mélange A avec de la boue primaire fraîche,
c) séparation de la boue primaire.

2. Procédé selon la revendication 1, **caractérisé en ce que** l'acide sulfurique concentré qui est utilisé pour l'acidification présente une concentration de 85 à 95 % en poids, de préférence de 90 à 93 % en poids.

3. Procédé selon la revendication 1 ou la revendication 2, **caractérisé en ce que** dans l'étape a) on utilisé la totalité de l'acide sulfurique rejeté qui a été produit lors de la nitration.

4. Procédé selon l'une quelconque des revendications 1 à 3, **caractérisé en ce que** dans l'étape a) on ajuste le pH à une valeur de 0,5 à 3.

5. Procédé selon l'une quelconque des revendications 1 à 4, **caractérisé en ce que** le mélange A et la boue primaire sont mis en contact l'un avec l'autre pendant 1 minute à 2 jours.

6. Procédé selon l'une quelconque des revendications 1 à 5, **caractérisé en ce que** pour ce qui concerne les composés aromatiques il s'agit du benzène, du toluène, du xylénol, du chlorobenzène et/ou du dichlorobenzène.

7. Procédé selon l'une quelconque des revendications 1 à 6, **caractérisé en ce que** l'eau résiduaire alcaline de processus dans l'étape a) et le mélange A au début de l'étape b) présentent une température de 50 à 80 °C, de préférence de 60 à 75 °C.

8. Procédé selon l'une quelconque des revendications 1 à 7, **caractérisé en ce que** la boue primaire présente au début de l'étape b) une température de 5 à 25 °C, de préférence de 8 à 15 °C.

9. Procédé selon l'une quelconque des revendications 1 à 8, **caractérisé en ce que** dans l'étape b) on choisit le rapport volumique de la boue primaire au mélange A de manière que s'ajuste une température de 30 °C ou moins.

10. Procédé selon l'une quelconque des revendications 1 à 9, **caractérisé en ce que** dans l'étape b) le rapport volumique de la boue primaire au mélange A vaut de 5:1 à 1:2, de préférence de 3:1 à 1:1.

11. Procédé selon l'une quelconque des revendications 1 à 10, **caractérisé en ce que** dans l'étape b) le mélange A et la boue primaire sont mis en contact à l'aide d'éléments de mélange actifs et/ou passifs.

12. Procédé selon l'une quelconque des revendications 1 à 11, **caractérisé en ce que** les oxydes d'azote qui se séparent lors de l'acidification dans l'étape a) sont isolés et utilisés pour l'emploi dans la production d'acide nitrique.

13. Procédé selon l'une quelconque des revendications 1 à 12, **caractérisé en ce que** les oxydes d'azote qui se séparent lors de l'acidification dans l'étape a) sont isolés et recyclés dans la récupération d'acide nitrique de l'unité de nitration.

14. Procédé selon l'une quelconque des revendications 1 à 13, **caractérisé en ce que** dans l'étape c) on sépare la boue primaire dans un séparateur statique.

15. Procédé selon l'une quelconque des revendications 1 à 14, **caractérisé en ce que** le procédé est mis en oeuvre en mode opératoire continu.
